Europäisches Patentamt

European Patent Office

Office européen des brevets

(19)

(11) Veröffentlichungsnummer: **0 246 526**
B1

# EUROPÄISCHE PATENTSCHRIFT

(12)

(45) Veröffentlichungstag der Patentschrift:
01.08.90

(21) Anmeldenummer: 87106778.1

(22) Anmeldetag: 11.05.87

(51) Int. Cl.⁵: **C07D 257/02, C06B 21/00**

(54) **Verfahren zur Herstellung von feinkörnigem Beta-Oktogen.**

(30) Priorität: 23.05.86 DE 3617408

(43) Veröffentlichungstag der Anmeldung:
25.11.87 Patentblatt 87/48

(45) Bekanntmachung des Hinweises auf die Patenterteilung:
01.08.90 Patentblatt 90/31

(84) Benannte Vertragsstaaten:
BE CH DE FR GB IT LI SE

(56) Entgegenhaltungen:
DE-A- 2 308 430
DE-A- 3 510 761
US-A- 3 297 681
US-A- 3 770 721

Die Akte enthält technische Angaben, die nach dem
Eingang der Anmeldung eingereicht wurden und die
nicht in dieser Patentschrift enthalten sind.

(73) Patentinhaber: Dynamit Nobel Aktiengesellschaft,
Postfach 12 61, D-5210 Troisdorf(DE)

(72) Erfinder: Heinemeyer, Klaus, Hornpottweg 52,
D-5090 Leverkusen 1(DE)
Erfinder: Redecker, Klaus, Dr., Burgfarrnbacher
Strasse 34, D-8500 Nürnberg(DE)
Erfinder: Sassmannshausen, Ulrich, Dr., Sechtemer
Strasse 6, D-5040 Brühle(DE)

**Beschreibung**

Gegenstand der vorliegenden Erfindung ist ein Verfahren zur Herstellung eines besonders feinkörnigen β-Oktogens, bei dem man aus einer β-Oktogen-Lösung die Kristalle mittels eines Nichtlösungsmittels für β-Oktogen ausfällt.

Der Explosivstoff Cyclotetramethylentetranitramin, der allgemein als Oktogen bezeichnet wird, existiert in vier verschiedenen Modifikationen, von denen nur die β-Form bei Temperaturen bis 104 °C stabil ist und damit - unter geeigneten Sicherheitsmaßnahmen - unbegrenzt lagerfähig ist. Bei seiner Herstellung fällt es in Korngrößen von ca. 50 μm ($10^{-6}$ m) an. Für bestimmte Anwendungszwecke wird β-Oktogen jedoch in Korngrößen unter 50 μm bis herab zu Korngrößen von 2 μm benötigt, so daß es notwendig ist, Verfahrensweisen aufzufinden, ein solches feinkörniges β-Oktogen herzustellen.

Die an sich naheliegende Zerkleinerung der Kristalle durch Mahlen stößt auf die Schwierigkeit, daß β-Oktogen äußerst reib- und stoßempfindlich ist, so daß bei einer mechanischen Zerkleinerung stets die Gefahr einer Explosion besteht. Die Zerkleinerung findet deshalb durch spezielle Mahlverfahren statt. Die dabei erhaltenen Kristalle haben jedoch den Nachteil, daß sie scharfe Bruchkanten aufweisen die wiederum die Sicherheitseigenschaften des Produkts beeinträchtigen.

In der US-PS 3 297 681 wird ein Verfahren zur Herstellung von feinkörnigem Oktogen beschrieben, das darin besteht, die Lösung von Oktogen in einem beliebigen organischen Lösungsmittel bei 20 bis 35°C mit ß-Oktogen-Kristallen anzuimpfen und einen Nichtlöser für Oktogen hinzuzufügen. Anschließend wird das Lösungsmittel abdestilliert und die erhaltenen Kristalle abgefiltert.

Es bestand deshalb die Aufgabe, ein Verfahren zur Herstellung von feinkörnigem ß-Oktogen mit Korngrößen unter 50 μm aufzufinden, bei dem die obengenannten Sicherheitsrisiken weitgehend ausgeschaltet sind und bei dem ein Produkt erhalten wird, das lagerfähig unter Beibehaltung der Korngröße ist.

In Erfüllung dieser Aufgabe wurde nun ein Verfahren zur Herstellung von ß-Oktogen durch Umkristallisieren einer ß-Oktogen enthaltenden Lösung mittels eines Nichtlösers für ß-Oktogen gefunden, das dadurch gekennzeichnet ist, daß man die Umkristallisation aus einer Lösung des ß-Oktogens in einem γ-Lacton mit Toluol im Temperaturbereich zwischen 5 und 15°C durchführt und anschließend die dabei erhaltenen Kristalle in an sich bekannter Weise weiter aufarbeitet.

In der DE 3 510 761-A1 wird zwar auch eine Verfahrensweise beschrieben, Oktogen aus Lacton-Lösungen auszufällen; das Fällungsmittel ist jedoch Wasser und man erhält Kristalle unterschiedlicher Größe aus α- und ß-Oktogen. Daneben entstehen Kristalle eines Butyrolacton-Komplexsalzes.

Bei Durchführung der erfindungsgemäßen Verfahrensweise erhält man ein β-Oktogen sehr großer Reinheit, dessen Gehalt an anderen Modifikationen des Oktogens, insbesondere der α–Form, unter 0,5% liegt und dessen mittlere Korngröße bis herab

zu 5 μm beträgt. Die Ausbeuten bei dieser Verfahrensweise liegen etwa bei 90%.

Das als Ausgangsprodukt eingesetzte β-Oktogen braucht nicht die hohe Reinheit in bezug auf andere Modifikationen des Oktogens zu besitzen. Selbst wenn es z.B. 5 % der α- oder einer anderen Modifikation enthält, wird ein Produkt der vorgenannten Reinheit erhalten.

Als Lösungsmittel für das β-Oktogen eignen sich prinzipiell alle bei Raumtemperatur flüssigen γ-Lactone, die ein Lösungsvermögen für β-Oktogen besitzen. Bevorzugtes Lacton ist das γ-Butyrolacton. Das Auflösen des β-Oktogens in den Lactonen zur Herstellung der bei dem erfindungsgemäßen Verfahren einzusetzenden Lösungen soll nach Möglichkeit bei Temperaturen bis zu 50 °C durchgeführt werden.

Die Ausfällung mit Toluol soll im Temperaturbereich zwischen 5 und 15 °C erfolgen. Bei dieser Temperatur werden besonders feine und sehr reine β-Oktogen-Kristalle erhalten, die im Korngrößenbereich um 5 μm liegen. Bei höheren Temperaturen ist der Anteil an größeren Kristallen höher oder es treten Modifikationsänderungen auf.

Eine bevorzugte Durchführungsform für die Einhaltung der Fällungstemperatur besteht darin, die β-Oktogen-Lösung in gekühltes Toluol der angegebenen Temperatur als Vorlage eintropfen zu lassen. Dabei soll das Toluol kräftig gerührt werden. Jedoch sind auch andere Durchführungsformen erfindungsgemäß möglich, wenn die Einhaltung des Temperaturbereichs von 5 bis 15 °C dabei gewährleistet ist.

Das Abtrennen und Trocknen der ausgefallenen Kristalle erfolgt auf an sich bekannte Weise. Die Trocknungstemperaturen sollten jedoch 50 °C nach Möglichkeit nicht überschreiten, da sonst die Gefahr des Zusammenbackens der Kristalle besteht.

Zur Verhinderung des Zusammenbackens der feinen Kristalle, die nach dem beanspruchten Verfahren erhalten werden, wurde eine weitere Durchführungsform des beanspruchten Verfahrens gefunden, die darin besteht, die erhaltenen Kristalle nach ihrer Abtrennung aus der Toluol-Lösung mit einer Umhüllung aus Kunststoffen zu versehen. Dabei wird im wesentlichen folgendermaßen vorgegangen:

Das abgetrennte β-Oktogen wird in Wasser aufgeschlämmt und bei Temperaturen zwischen 25 und 60 °C unter Rühren mit einer Lösung oder Emulsion oder Suspension eines thermoplastischen Polymeren versetzt, wobei Granulatbildung erfolgt; anschließend wird die erhaltene Granulat-Dispersion auf Temperaturen bis zu maximal 100 °C erhitzt, um das Lösungsmittel für das Polymere abzudestillieren. Das dann erhaltene Granulat hat eine erheblich geringere Stoß- und Reibempfindlichkeit als nicht umhülltes β-Oktogen. Es ist gut rieselfähig, und die umhüllten Kristalle neigen nicht zur Agglomeration oder Klumpenbildung, wie es bei nicht umhüllten Kristallen leicht der Fall sein kann.

Als thermoplastische Polymere für diese Umhüllung eignen sich Polyvinylacetale, die durch Umsetzung von Polyvinylalkohol mit Aldehyden erhalten werden, wie z.B. Polyvinylbutyral. Auch Acrylharze sind einsetzbar, wie z.B. Methylacrylat-, Methyl-methacrylat- oder Acrylnitrilharze. Bevorzugtes Harz ist das Polyvinylbutyralharz. Die Umhüllung mit dem Harz macht zwischen 3 und 15 Gew.-% der umhüllten Kristalle aus.

Beispiel 1

Es wurden 20 g eines handelsüblichen β–Oktogens einer durchschnittlichen Korngrösse von 55 μm in 100 ml γ-Butyrolacton bei etwa 30°C gelöst. Diese Lösung wurde langsam in eine Vorlage aus Toluol eingerührt, wobei Ausfällung eintrat; der erhaltene Niederschlag wurde abfiltriert, mit Wasser gewaschen und bei Temperaturen unter 50°C getrocknet. Die Ausbeute lag bei 90%. Die mittlere Korngröße des erhaltenen Produkts war < 6 μm, gemessen mit dem Sedimentograph "Analysette 20".

Beispiel 2

Als Ausgangsprodukt wurden 40 g eines β-Oktogens einer mittleren Korngröße von 55 μm eingesetzt, dessen Gehalt an γ-Oktogen bei 2% lag. Die Verarbeitung erfolgte analog Beispiel 1. Das erhaltene β-Oktogen hatte ebenfalls eine mittlere Korngröße < 6 μm gemessen als Dispersion in Wasser mit dem "Analysette 20". Der Gehalt an γ-Oktogen Sedimentograph lag gemäß IR-Spektrometrie unter 0,5 Gew.-%.

**Patentansprüche**

1. Verfahren zur Herstellung von feinkörnigem β-Oktogen durch Umkristallisation einer β-oktogenhaltigen Lösung mittels eines Nichtlösers für β-Oktogen, dadurch gekennzeichnet, daß man die Umkristallisation aus einer Lösung des β-Oktogens in einem γ-Lacton mit Toluol im Temperaturbereich zwischen 5 und 15 °C durchführt und anschließend die dabei erhaltenen Kristalle in an sich bekannter Weise weiter aufarbeitet.

2. Verfahren gemäß Anspruch 1, dadurch gekennzeichnet, daß man die β-Oktogen-Lösung einer Vorlage von Toluol zuführt.

3. Verfahren gemäß Anspruch 1 oder 2, dadurch gekennzeichnet, daß man als γ-Lacton γ-Butyro-lacton einsetzt.

4. Verfahren gemäß einem der Ansprüche 1 bis 3, dadurch gekennzeichnet, daß man die erhaltenen Kristalle nach ihrer Abtrennung mit einer Kunststoffumhüllung versieht.

**Claims**

1. Process for the production of finely particulate β-octogen by recrystalization from a β-octogen containing solution by means of a non-soluent for β-octogen, characterised in that the recrystalization is carried out from a solution of the β-octogen in a lγ-actone by means of toluene in the temperature range between 5 and 15°C and then the crystals obtained therein are worked up further in known manner.

2. Process according to claim 1, characterised in that the β-octogen solution is supplied to a recipient vessel of toluene.

3. Process according to claim 1 or 2, characterised in that γ-butyro-lactone is employed as γ-lactone.

4. Process according to 1 of claims 1 to 3, characterised in that the crystals obtained are provided with a plastics envelope after their separation off.

**Revendications**

1. Procédé pour la préparation de β–octogène à grains fins, par recristallisation d'une solution contenant du β-octogène, au moyen d'un non-solvant pour le β-octogène, caractérisé en ce que l'on effectue la recristallisation à partir d'une solution du β-octogène dans une γ-lactone, avec du toluène, dans la plage de températures comprise entre 5 et 15°C, et on poursuit ensuite d'une façon connue en soi le traitement des cristaux ainsi obtenus.

2. Procédé selon la revendication 1, caractérisé en ce que l'on introduit la solution de β-octogène dans un récipient contenant du toluène.

3. Procédé selon la revendication 1 ou 2, caractérisé en ce que, en tant que γ-lactone, on utilise de la γ-butyrolactone.

4. Procédé selon l'une des revendications 1 à 3, caractérisé en ce que, après leur séparation, on munit d'un enrobage de matière plastique les cristaux obtenus.